(19) Europäisches Patentamt
European Patent Office
Office européen des brevets

(11) Publication number: **0 153 001 B1**

(12) **EUROPEAN PATENT SPECIFICATION**

(45) Date of publication of patent specification: **11.03.92**

(51) Int. Cl.[5]: **C07J 41/00**, C07J 43/00, C07J 7/00

(21) Application number: **85300115.4**

(22) Date of filing: **08.01.85**

(54) **Steroids having an enamide or enimide group and their preparation.**

(30) Priority: **03.02.84 US 576590**
**03.02.84 US 576674**

(43) Date of publication of application:
**28.08.85 Bulletin 85/35**

(45) Publication of the grant of the patent:
**11.03.92 Bulletin 92/11**

(84) Designated Contracting States:
**BE CH DE FR GB IT LI NL SE**

(56) References cited:
**GB-A- 2 079 756**
**GB-A- 2 086 907**

**CHEMICAL ABSTRACTS, vol. 97, no. 23, 6th December 1982, pages 603,604, no. 198455w, Columbus, Ohio, US; & JP - A - 82 88 197 (MITSUBISHI CHEMICAL INDUSTRIES CO., LTD.) 01-06-1982**

(73) Proprietor: **THE UPJOHN COMPANY**
**301 Henrietta Street**
**Kalamazoo, Michigan 49001(US)**

(72) Inventor: **VanRheenen, Verlan Henry**
**c/o The Upjohn Company 301 Henrietta Street**
**Kalamazoo Michigan 49001(US)**

(74) Representative: **Perry, Robert Edward et al**
**GILL JENNINGS & EVERY 53-64 Chancery Lane**
**London WC2A 1HN(GB)**

**EP 0 153 001 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 97, no. 19, 8th November 1982, page 750, no. 163318b, Columbus, Ohio, US; D.H.R. BARTON et al.: "A simple construction of the corticosteroid side chain from 17-ketosteroids", & NOUV. J. CHIM. 1982, 6(6), 295-300

JOURNAL OF THE CHEMICAL SOCIETY, CHEMICAL COMMUNICATIONS, no. 15, 1981, pages 774,775, London, GB; D.H.R. BARTON et al.: "Efficient synthesis of the corticosteroid side-chain from 17-ketones"

JOURNAL OF THE CHEMICAL SOCIETY, PERKINS TRANSACTIONS I, 1975, pages 1242-1244, London, GB; R.B. BOAR et al.: "A new and convenient synthesis of the 17-alpha, 21-diacetoxy-20-oxopregnane side-chain"

JOURNAL OF THE AMERICAN CHEMICAL SOCIETY, vol. 81, no. 21, 5th November 1959, pages 5725,5727; P. DE RUGGIERI et al.: "17-Hydroxypregnanes from androstane compounds"

TETRAHEDRON LETTERS no. 22, 1971, pages 2005-2008, Pergamon Press, Oxford, GB; J.C. GASC et al.: "A new approach to corticoid total synthesis"

## Description

This invention relates to steroids having an enamide, enimide or cyano group, their preparation and their use.

Processes for transforming 17-keto steroids to the corresponding 17α-hydroxpregna steroids are disclosed in, for example, US-A-4041055 and Tet. Lett. 22 (1971) 2005. Processes for transforming 17-keto steroids to the corresponding corticoids are disclosed in US-A-4041055, US-A-4342702, US-A-4216159 and GB-A-2086907.

Processes for transforming a 17β-cyano-17α-hydroxy steroid (I) to the corresponding 17α-acyloxyprogesterone (VI) are disclosed by deRuggieri et al., J.A.C.S. 81 (1959) 5725; Gasc et al., Tet. Lett. (1971) 2005; Belanger, Steroids 37 (1981) 361; JP-A-7062296, JP-A-7062297, JP-A-7062299 and JP-A-7062300.

17β-Cyano-17α-hydroxy steroids (I) have been produced from the corresponding 17-keto steroids, as described by Ercoli et al., J.A.C.S. 75 (1953) 650; Heusser et al., Helv. Chem. Acta 33 (1950) 1093; Meyer, Helv. Chem. Acta 29 (1946) 1580; Miescher et al., Helv. Chem. Acta 21 (1938) 1317; Kuhl et al., Steroids 28 (1976) 89; US-A-3496169 and DD-A-147669. JP-A-7062296, JP-A-7062299 and JP-A-7062300 also disclose 17β-cyano-17α-hydroxyandrosta-4,9(11)-dienes (IA). Gasc et al., supra reported the preparation of 19-nor-17β-cyano-17α-hydroxyandrosta-5(10),9(11)-dienes.

17β-Cyano-17α-hydroxy steroids (I), in which the 17α-hydroxy group is protected as an ester, are known as described in, for example, Helv. Chem. Acta 29 (1946) 1582 (formula IV), and Helv. Chem. Acta 33 (1950) 1096 (formula XIII). These same steroids, in which the 17α-hydroxy group is protected as an ether, are also known, as described in, for example, Steroids 37 (1981) 362 (the TMS ether); deRuggieri et al., supra, at p.5726 (formula III as the THP ether); Gasc et al, supra, on p.2007 (formula IX as the TMS ether); and JP-A-7062296 for the butyl vinyl ether.

US-A-4348327 discloses 17β-cyano-17α-hydroxy steroids of the $\Delta^4$-3-keto (A) and 3β-hydroxy-$\Delta^5$ (C) types, with or without substitution at $C_{16}$, which have no substitution in the C ring.

Barton et al., J.C.S. Chem. Comm. (1981) 774, reported producing a 3β-O-substituted-$\Delta^5$-enimide similar to the enimide (III) where the protecting group at 17α was acetate and the substituent on the enimide nitrogen at $C_{20}$ was -CHO. Barton et al obtained the 3β-O-substituted-$\Delta^5$-enimide from a 17(20)-unsaturated steroid.

R.B. Boar et al, J.C.S. Perkin I 1242 (1975), reported a 3β-acetoxy-$\Delta^5$-20-acetyl enimide, obtained from a pregnenolone derivative. These compounds are somewhat similar to the enimide (III), but include, at the 17α position, an ester protecting group that has the disadvantage of being difficult to remove.

Boar et al, supra, also disclose a $\Delta^{20}$-enimide acylate, obtained from the corresponding 3β-acetyl-$\Delta^5$-enimide by reaction with acetic acid containing trichloroacetic acid. The product had no substitution in the C-ring.

Boar et al, supra, also disclose a $\Delta^{20}$-enimide (see compound IV on p. 1243) including an ester group at $C_{17}$.

Barton et al, supra, also disclose a $\Delta^{20}$-enimide 17-acetate (see compound 5) that has, as a substituent on the enamide nitrogen atom, an aldehyde (a formyl group).

Boar et al, supra, disclose the transformation of an enimide-17-ester to a $\Delta^{16}$-progesterone, and the hydrolysis of the enimide 17-acetate to a 20-keto-17-acetate (VI).

The oxazoline (IX) structure type is known; see GB-A-2086907 and Barton et al, supra. Barton et al disclose transforming a $\Delta^{17(20)}$-20-amide to the oxazoline by reaction with a peracid, and propose a reaction mechanism involving a 17,20-epoxide and a 17α-hydroxy-2-enimide. However, none of the intermediates was isolated or identified, nor was the mechanism proved.

Barton et al, supra, further disclose the transformation of a $\Delta^{20}$-enamide (5), where the nitrogen was substituted with a formamide, to the 21-bromo enimide (6), wherein both contain the 17-acetate. They also disclose transformation of the 21-bromo steroid (7) to the 21-bromo-20-keto steroid (8), a process in which the ester at C-17 was not lost.

## Summary of the Invention

The compounds of formulae III A-C and V A-C, which are tautomeric, are novel. They are protected at C-17 by an ether group, unlike the compounds disclosed by Boar et al.

A first novel process, for the preparation of a $C_3$-protected form of an enimide (III A-C), comprises (1) contacting a $C_3$-protected form of a 17-protected-17β-cyano-17α-hydroxy steroid (II A-C) with a methylating agent and (2) contacting the product of step (1) with an acylating or silating agent. By contrast with Boar's procedure, the novel process has the flexibility to produce the enimide (III) in which the C-17 OH group is

protected with an ether.

A second novel process, for the preparation of a $C_3$-protected form of a $\Delta^{20}$-enamide acylate (IV A-C), comprises contacting a $C_3$-protected form of an enimide (III A-C) or of a $\Delta^{20}$-enamide (V A-C) with a carboxylic acid of the formula $R_{17}COOH$ and an anhydride of the formula $(R_{17}CO)_2O$, where $R_{17}$ is H, $C_{1-5}$ alkyl, $CCl_3$, $CF_3$ or phenyl substituted by 0 to 2 Cl. The product can be converted to commercial corticoids more easily than the compounds disclosed by Boar et al.

A third novel process, for the preparation of a $C_3$-protected form of a $\Delta^{20}$-enamide (V A-C), comprises contacting a $C_3$-protected form of an enimide (II A-C) with an acid or base.

A fourth novel process, for the preparation of a $C_3$-protected form of an oxazoline (IX A-C), comprises contacting a $C_3$-protected form of an enimide (III A-C) or of a $\Delta^{20}$-enamide (V A-C) with an acid in an organic solvent.

A fifth novel process, for the preparation of $C_3$-protected forms of a 21-halo enimide (XV A-C) and a 21-halo-$\Delta^{20}$-enamide (XX A-C), comprises contacting a $C_3$-protected form of a $\Delta^{20}$-enamide (V A-C) with a halogenating agent. The products can be converted to a $C_3$-protected form of a 17α-hydroxy steroid (XIII A-C) by reaction with an aqueous acid. By contrast with Barton's preparation, the free 17α-hydroxy compound is produced.

## Detailed Description of the Invention

17-Keto steroids, from which compounds of the invention can be prepared, are well known to those skilled in the art or can readily be prepared from known compounds by methods well known to those skilled in the art. These include $\Delta^4$-3-keto (A), $\Delta^{1,4}$-3-keto (B) and 3β-hydroxy-$\Delta^5$ (C) steroids, see Chart F. The 17-keto starting materials can be substituted at $C_6$, $C_9$, $C_{11}$ and/or $C_{16}$, with $R_6$, $R_9$, $R_{11}$ and $R_{16}$ as defined infra.

The A-ring of the 17-keto starting material does not have to be protected to form the 17β-cyano-17α-hydroxy steroid (I). However, during the methylation reaction of transforming the 17-protected-17β-cyano-17α-hydroxy steroid (II) to the enimide (III) the A-ring must be protected.

For the $\Delta^4$-3-keto steroids (A) the $C_3$ ketone is protected as the enol ether (Aa), ketal (Ab), or enamine (Ac) as is well known in the art, see Chart G. The preferred enol ether (Aa) is the methyl or ethyl ether. The preferred ketal (Ab) is the ethylene ketal. The preferred enamines are selected from the group consisting of pyrrolidine, morpholine and diethylamino amines. The enol ethers (a) are prepared by methods well known in the art, see J. Org. Chem. 26, 3925 (1961), Steroid Reactions, Edited by Carl Djerassi, Holden-Day, San Francisco, 1962, p. 42-45, and U.S. Patent 3,516,991 (Preparation 1). The ketals (b) are also prepared by well known methods, see Steroid Reactions, supra,, p. 11-14. The 3-enamines (c) are also prepared by methods well known in the art, see U.S. Patent 3,629,298 and Steroid Reactions, supra, p. 49-53.

The $\Delta^{1,4}$-3-keto steroids (B) are protected by reacting the 17-protected-17β-cyano-17α-hydroxy steroid (II) with a strong base such as LDA (lithium diisopropyl amide) to form an enolate represented by formula (Ba). The enolate is then methylated and acylated or silated to form the enimide (III). In the process the 3-enolate is also acylated or silated to give the 3-acylate or 3-silyl derivative of (Ba). In a subsequent workup and hydrolysis the A-ring enolate reforms the $\Delta^{1,4}$-3-keto (B) A-ring functionality.

The 3-hydroxy steroid (C) should have the 3β-hydroxyl group protected as the ether (Ca), see Chart G.

The $C_3$ protected forms (Aa, Ab and Ac) of the $\Delta^4$-3-keto steroids (A), the $C_3$ protected forms (Ba) of the $\Delta^{1,4}$-3-keto steroids (B) and the $C_3$ protected forms (Ca and Cb) of the 3β-hydroxy steroids (C) are considered equivalent to the non-protected or free form (A, B and C) respectively since the $C_3$ protecting groups are readily removable to convert the $C_3$ protected forms (Aa, Ab, Ac, Ba and Ca) to the free or unprotected forms (A, B and C) respectively. In order to remove the $C_3$ protecting group from the $\Delta^4$-3-keto steroids (A), $\Delta^{1,4}$-3-keto steroids (B) and 3β-hydroxy steroids (C) the reaction conditions would generally also remove the $C_{17}$ protecting group (Z) which is undesirable. Therefore, the $C_3$ protecting group remains during subsequent reactions not because it is needed per se but rather so as not to remove the (Z) protecting group. The $C_3$ and $C_{17}$ protecting groups can readily be removed, if desired, from the intermediates producing 17α-hydroxy-free-A-ring steroids.

The 17-keto steroid starting material is reacted with potassium cyanide and 2-cyano-2-hydroxypropane (acetone cyanohydrin) in an alcoholic solvent such as aqueous methanol to give the desired 17β-cyano-17α-hydroxy steroid (I) with high yields and stereospecificity as is known in the art. The reaction may be heated to 30-50° if desired.

Before protecting the 17α-hydroxy group of the cyanohydrin (I), it is preferred that the $C_3$ function of the $\Delta^4$-3-keto (A), $\Delta^{1,4}$-3-keto (B) or 3β-hydroxy-$\Delta^5$ (C) steroids be protected as discussed supra although the 17α-hydroxy group may be protected first. 17β-Cyano-17α-hydroxy steroids (I) are known where the 17α-hydroxy group is protected as an ether. See, Steroids 37, 362 (1981) on p 362 for the TMS ether; J. Am. Chem. Soc. 81, 5725 (1959) on p 5726 formula III as the THP ether; Tetra. Lett. 22, 2005 (1971) on p 2007 formula IX as the TMS ether and Japanese Patent 7,062,296 for the butyl vinyl ether. The preferred 17α-protecting group is the ethoxy ethyl ether. Other suitable groups include THP, TMS, methoxymethyl, and butoxy ethyl ethers. The appropriate form of the 17β-cyano-17α-hydroxy steroid (I) is reacted with a reagent to form the desired ether protecting group (Z) such as ethoxy ethyl ether (EEE) using an acid catalyst such as pyridine hydrochloride, p-TSA, or in an inert solvent. Suitable solvents include toluene, SSB, cyclohexane, methylene chloride.

The enimide (III) and the $\Delta^{20}$-enamide (V) are tautomers. The 17-protected-17β-cyano-17α-hydroxy steroid (II) can be converted to both the enimide (III) and the $\Delta^{20}$-enamide (V). The 17-protected-17β-cyano-17α-hydroxy steroid (II) is converted to the enimide (III) by first reaction with a methylating agent. Addition of the methylating agent to the protected cyanohydrin (II) gives an intermediate imine which can be isolated, but it is preferred to acylate or silate the imine anion in situ by addition of an acylating or silating agent. The acylating agents are selected from the group consisting of acyl halides and acyl anhydrides of the formulas $R_{20}J$, $(R_{20})_2O$, or $R_{20}$"J. Suitable solvents for this reaction include ethers such as THF. The reaction is performed in the temperature range of about -30° to about 100°. Silating agents include J-Si-$(CH_3)_3$ and J-$Si(CH_3)_2C(CH_3)_3$.

Some methylating agents have greater Lewis acid properties or form better Lewis acids after methylation than other methylating agents. For example, methyl Grignard is a better Lewis acid than methyl lithium. Methylating agents such as methyl Grignard having sufficient Lewis acidity transform the initially formed enimide (III) to its tautomeric form the $\Delta^{20}$-enamide (V). Thus, with the methylating agents which are Lewis acids, such as methyl magnesium chloride the ratio of tautomeric products, the enimide (III) and $\Delta^{20}$-enamide (V) is time dependent. At short time periods the predominate product is the enimide (III). With time there is a decrease in the amount of enimide (III) and increase the amount of $\Delta^{20}$-enamide (V) to the point where predominately the $\Delta^{20}$-enamide (V) is present. Methylating agents which are poor Lewis acids include, for example, methyl lithium. The 17-protected -17β-cyano-17α-hydroxy steroid (II) is transformed to the enimide (III) if the methylating agent is methyl lithium and the imine anion formed is acylated or silated. Methylating agents which are or produce Lewis acids include Grignard reagents, for example, methyl magnesium bromide, chloride and iodide. For example, if the methylating agent is methyl magnesium bromide and the acylating agent is acetic anhydride, during the methylation/acylation step of transforming the 17-protected-17β-cyano-17α-hydroxy steroid (II) to the enimide (III) the methyl magnesium bromide is transformed to magnesium bromoacetate which is a (Lewis) acid and therefore the enimide (III) which is initially produced is transformed to the $\Delta^{20}$-enamide (V) without addition of another acid, see Example 7. If it is desired to isolate the enimide (III) the preferred methylating agent utilized in the methylation reaction is one that in situ will not be transformed to a Lewis acid. Methyl lithium is a methylating agent operable in effectuating the methylation reaction but will not form a Lewis acid and will permit isolation of the enimide (III), see Examples 5 and 18. The preferred methylating reagent is methyl lithium.

The enimide (III) is isomerized to its tautomer the $\Delta^{20}$-enamide (V) by reaction with an acid, or a base. Acids include Lewis acids, and carboxylic acids including polycarboxylic acids. It is preferred that the acid be acetic, propionic, benzoic or citric. The most preferred acid is acetic acid. Bases which are operable include guanidine and amidine bases, preferred are DBU and DBN.

The enimide (III) and $\Delta^{20}$-enamide (V) are tautomers. Both can be transformed to corresponding $\Delta^{20}$-enamide 17-ester (IV) by reaction with a carboxylic acid of the formula $R_{17}COOH$ and a carboxylic acid anhydride of the formula $(R_{17}CO)_2O$ in the acid as solvent in the temperature range of about 0 to about 80° preferably at about 20° to about 25°. The reaction converts the protecting group "Z" at C-17 to the 17-ester ($-OCOR_{17}$) and with the enimide (III) also the enimide portion is tautomerized to the $\Delta^{20}$-enamide. It is preferred that $R_{17}$ is methyl, ethyl or phenyl, more preferably methyl. If $R_{17}$ is a hydrogen atom, trichloromethyl, trifluoromethyl or an equivalent substituent which makes the ester $R_{17}COO$- active, such ester (IV) may be selectively removed with mild base such as bicarbonate in methanol to give the 17α-hydroxy-$\Delta^{20}$-enamide (XVII). Likewise the 21-halo steroid (XI) can be converted to the 21-halo-17α-hydroxy corticoid (XIII). Alternatively, if $R_{17}$ is trimethyl silyl or other convenient silyl protecting group, such group may be removed with mild base as above, mild acid or fluoride ion producing the desired 17α-hydroxyl group in XVII.

The $\Delta^{20}$-enamide (V) can be transformed to the 17α-hydroxyprogesterone 17-ester (VI) by reaction with an appropriate esterifying agent such as an anhydride $(R_{17}CO)_2O$ and the corresponding carboxylic acid $R_{17}COOH$ to produce the enamide acylate (IV) followed by addition of water to hydrolyze the enamide acylate (IV) to the 17α-hydroxyprogesterone 17-ester (VI).

The enimide (III) can be readily transformed to a 17α-hydroxyprogesterone 17-ester (VI) by reaction

with the appropriate esterifying agent such as an anhydride $(R_{17}CO)_2O$ or acid halide $R_{17}COJ$ in the corresponding acid, $R_{17}COOH$, solvent. Preferred is acetic anhydride in acetic acid followed by water. This in situ acetylation avoids an often difficult separate acylation step on the hindered tertiary 17α-hydroxy group, see U.S. Patent 4,154,748. If one were to start with androstenedione as the 17-ketone and perform the process of the present invention using acetic anhydride/acetic acid in converting the enimide (III) or $\Delta^{20}$-enamide (V) to the $\Delta^{20}$-enamide acylate (IV), one would obtain on hydrolysis 17α-hydroxyprogesterone 17-acetate (VI'A) as the 17α-hydroxyprogesterone 17-ester (VI), see Chart B. The 17α-hydroxyprogesterone 17-acetate (VI'A) can be transformed to 17α-hydroxy-6-methyleneprogesterone 17-acetate (VII) by the process of U.S. Patent 3,642,840, Example 11 which can be transformed to 17α-hydroxy-6α-methylprogesterone 17-acetate (VIII) which is medroxyprogesterone by the process of U.S. Patent 3,679,715, Example 1.

Alternatively, by starting with 6α-methylandrost-4-ene-3,17-dione (U.S. Patent 3,166,551, Example 8) as the 17-keto steroid and performing the process of the present invention using acetic anhydride/acetic acid as the acetylating agent, 17α-hydroxy-6α-methylprogesterone 17-acetate (VIII) is obtained directly.

Just as the tautomers, the enimide (III) and $\Delta^{20}$-enamide (V) can both be transformed to the corresponding $\Delta^{20}$-enamide 17-ester (IV), likewise they both can readily be converted to the oxazoline (IX) by contacting the tautomer with an acid in an organic solvent. Mineral, Lewis, or organic acids are suitable. This would include sulfuric, hydrochloric, phosphoric and equivalent inorganic acids. Organic acids such as p-TSA and carboxylic ($R_{17}$-COOH) and dicarboxylic acids such as oxalic acid also are operable and are preferred. Suitable organic solvents include, for example, toluene, methylene chloride, ethyl acetate, methanol, ethanol, THF, diethyl ether, and mixtures thereof. The enimide (III) or $\Delta^{20}$-enamide (V) is contacted with the acid in the suitable organic solvent in a temperature range of about 20° to about 60°. The reaction mixture can be heated to the reflux temperature of the particular solvent used. The reaction is monitored by TLC and when complete the oxazoline (IX) is isolated by means well known to those skilled in the art or can be halogenated without isolation.

The oxazoline (IX) is a very useful intermediate in the production of corticoid diesters (XII), see Chart C. The oxazoline (IX) is halogenated to produce the halo-oxazoline (X) according to the procedure set forth in J.C.S. Chem. Comm. 774 (1981). The halo-oxazoline (X) is then readily converted by hydrolysis to the 21-halo steroid (XI) which is readily transformed to the pharmacologically useful corticoid diester (XII). Alternatively, the 21-halo enimide (XV) and 21-halo-$\Delta^{20}$-enamide (XX) is readily converted into halo-oxazoline (X) by contacting with an acid in an organic solvent.

The $\Delta^{20}$-enamide (V) can be readily converted to the corresponding 21-halo enimide (XV) by reaction with a halogenating agent selected from the group consisting of bromine, pyridine hydrobromide perbromide, NBS, dibromantin and NCS. The 21-halo enimide (XV) can be converted into its tautomer, 21-halo-$\Delta^{20}$-enamide (XX) under the influence of acid or base. Acids include organic carboxylic acids or polycarboxylic acids either as the solvent or in suitable organic solvents such as THF, toluene or methylene chloride. Acids may include Lewis acids such as $BF_3$˜etherate in organic solvent. Bases which are operable include amidine bases. Preferred are DBU and DBN. Under certain conditions the halogenation reaction becomes sufficiently acidic or basic to cause tautomerization of the 21-halo enimide (XV) to the 21-halo-$\Delta^{20}$-enamide (XX).

The 21-haloenimide (XV) and/or the 21-halo-$\Delta^{20}$-enamide (XX) can be hydrolyzed under acidic conditions to either the 21-halo steroid (XI) or the 21-halo-17α-hydroxy corticoid (XIII) depending on the conditions chosen. To prepare the corticoid diester (XII), the 21-haloenimide (XV) or its tautomer 21-halo-$\Delta^{20}$-enamide (XX) is first hydrolyzed with relatively high concentrations of aqueous acid with an organic cosolvent. The acids include mineral acids and carboxylic acids such as sulfuric, acetic, hydrochloric, etc. Organic cosolvents include methanol, toluene, methylene chloride. The 21-halo steroid (XI) is then converted to the corticoid diester (XII) by treatment with acylate ion in an organic solvent. The preferred conditions are treatment of the 21-halo steroid (XI) with potassium acylate in a mixture of DMF and toluene at 50-100° for 6-18 hr. Specifically, to prepare the 17,21-diacetate (XII) the 21-halo steroid (XI) is treated with potassium acetate in DMF and toluene at 75° for 18 hr. The corticoid diesters (XII) are important pharmaceuticals, such as betamethasone dipropionate and diflorasone diacetate, and important intermediates such as Reichstein's Compound S 17,21-diacetate which is converted to hydrocortisone 17,21-diacetate by fermentation. The corticoid diesters (XII) can be converted to the commercially important 17,21-dihydroxy corticoids by base hydrolysis. For example, hydrocortisone diacetate formula XII where $R_{11}$ is β-hydroxyl, $R_6$, $R_9$ and $R_{16}$ are hydrogen atoms can be converted to hydrocortisone by treatment with methanolic potassium carbonate at 20-25°. Hydrocortisone can be converted to hydrocortisone 21-acetate by reaction with acetic anhydride in pyridine as is well known to those skilled in the art.

To prepare the commercially important corticoid 21-ester (XIV) directly from the 21-halo-$\Delta^{20}$-enamide (XX) or the 21-halo enimide (XV) the 21-halo compounds are hydrolyzed with dilute aqueous inorganic

mineral acids such as sulfuric, hydrochloric or phosphoric in an organic solvent. The preferred conditions include treatment of (XV) or (XX) with dilute aqueous sulfuric acid in THF (Example 17) producing the 21-halo-17α-hydroxy corticoid (XIII). The 21-halo-17α-hydroxy corticoid (XIII) is converted to the corticoid 21-ester (XIV) by treatment with acylate ion as is well known to those skilled in the art.

## DEFINITIONS

The definitions and explanations below are for the terms as used throughout the entire patent application including both the specification and the claims.

All temperatures are in degrees Centigrade.

TLC refers to thin-layer chromatography.

IR refers to infrared spectroscopy.

UV refers to ultraviolet spectroscopy.

CMR refers to C-13 magnetic resonance spectroscopy, chemical shifts are reported in ppm (δ) downfield from TMS.

NMR refers to nuclear (proton) magnetic resonance spectroscopy, chemical shifts are reported in ppm (δ) downfield from TMS.

TMS refers to tetramethylsilane.

MS refers to mass spectrometry expressed as m/e or mass/change unit.

When solvent pairs are used, the ratio of solvents used are volume/volume (v/v).

THF refers to tetrahydrofuran.

DMF refers to dimethylformamide.

SSB refers to an isomeric mixture of hexanes.

p-TSA refers to p-toluenesulfonic acid monohydrate.

Hal is a chlorine, bromine, iodine, atom.

NBS refers to N-bromosuccinimide.

NCS refers to N-chlorosuccinimide.

TMS refers to trimethylsilyl (protecting group).

THP refers to tetrahydropyranyl.

EEE refers to ethoxyethyl ether.

DBU refers to 1,8-diazabicyclo[5.4.0]undec-7-ene.

DBN refers to 1,6-diazabicyclo[3.4.0]non-5-ene.

LDA refers to lithium diisopropylamide.

MEM refers to 2-methoxyethoxymethyl ($CH_3OCH_2CH_2OCH_2O$-).

Androstenedione refers to androst-4-ene-3,17-dione.

Any alkyl group defined herein includes isomers thereof where such exist.

R is H or $C_{1-5}$ alkyl; the R's can be the same or different.

$R_3$ is $C_{1-5}$ alkyl, with the proviso that, for the ketal (Ab) and the enamine (Ac), the $R_3$ groups can be connected and, when connected, may be connected by an oxygen or nitrogen atom.

$R_6$ is H, F or $CH_3$.

either $R_9$ is absent, $R_{11}$ is H, and the C-ring is $\Delta^{9(11)}$; $R_9$ and $R_{11}$ together form a 9β,11-epoxide; or $R_9$ is H or F and ...$R_{11}$ is -H, ~OH or =O;

$R_{16}$ is H or $CH_3$;

$R_{17}$ is H, $CCl_3$, $CF_3$, $C_{1-5}$ alkyl or phenyl substituted by 0 to 2 Cl atoms.

$R_{17}'$ is H or $CF_3$.

$R_{20}$ is -OC-$R_{20}'$ or -$R_{20}''$.

$R_{20}'$ is $C_{1-5}$ alkyl, phenyl substituted with 0 to 2 chlorine atoms, methyl or nitro groups.

$R_{20}''$ is -$Si(CH_3)_3$ or -$Si(CH_3)_2C(CH_3)_3$.

$R_{21}$ is $C_{1-5}$ alkyl or phenyl.

$R_{100}$ is $C_{1-3}$ alkyl.

J is a chlorine, bromine or iodine atom.

M is a lithium, sodium, potassium, magnesium or calcium atom.

Q is H or $C_{1-5}$ alkyl.

W is a TMS, THP or EEE group.

X refers to a hydrogen atom or nothing.

Z is TMS, THP, methoxymethyl, t-butyl-dimethyl silyl or EEE.

Z' is H or Z.

~ indicates that the attached atom or group can be in either the α or β configuration.

.... is a single or double bond.

The following Examples illustrate how to prepare the various compounds and/or perform the various processes of the invention.

Example 1 17β-Cyano-17α-hydroxyandrost-4-en-3-one (IA)

Acetone cyanohydrin (82 ml), potassium cyanide (1 g) and water (2 ml) are added to a slurry of androst-4-ene-3,17-dione (0.80 g) in methanol (400 ml) and water (40 ml) at 35°. The reaction becomes homogeneous and, on continued stirring, a heavy slurry develops. After 3 hours, 120 ml of water is added dropwise, and the slurry allowed to stir overnight at 20-25°. The slurry is then cooled in an ice-bath and filtered, and the solids washed with methanol/water (1/1). The solvents are dried under reduced pressure, to give 70.4 g of the title compound. An additional 13.17 g of product is isolated from the mother liquor on partial evaporation, m.p. = 159-160.5°; $[\alpha]^D$(c=1, $CHCl_3$) = + 145.9°; MS = 313, 298, 286, 245 and 124 m/e; NMR ($CDCl_3$) = 1.00, 1.20, 3.5 and 5.72 δ

Example 2 17β-Cyano-17α-hydroxyandrosta-4,9(11)-dien-3-one (IA)

Acetone cyanohydrin (5 ml) and sufficient potassium cyanide to adjust the pH to 9.9 is added to a slurry of androst-4,9(11)-dien-3,17-dione (0,5 g), methanol (40 ml) and water (2.5 g) at 40°. The reaction becomes homogeneous followed by precipitation of the product. After 3.5 hours, water (7.5 ml) is added dropwise and the reaction is allowed slowly to stir at 20-25° overnight. The slurry is cooled on in ice bath, filtered, the solids washed with methanol/water (1/1) and dried under reduced pressure to give the title compound (4.54 g); $[\alpha]^D$(c=1, $CHCl_3$) = +118°; MS = 311, 296 and 284 m/e; NMR ($CDCl_3$) = 0.97, 1.37, 2.9, 5.5 and 5.70 δ

Example 3 17β-Cyano-17α-hydroxyandrost-5-en-3-ethylidene ketal (IAb)

p-TSA (100 mg) is added to a mixture of 17β-cyano-17α-hydroxyandrost-4-en-3-one (IA, Example 1, 5 g) in methylene chloride (50 ml), ethylene glycol (5 ml) and trimethylorthoformate (2.5 ml). The resulting mixture is stirred 2 hours at 20-25°. The reaction mixture is concentrated to approximately 20 ml at atmospheric pressure. Triethylamine (0.2 ml) is added, the mixture extracted with phosphate buffer (pH 7) and the organic phase concentrated to a solid. The solid is crystallized from methanol to give the title compound (5.1 g); m.p. = 229.5-230.5°; $[\alpha]^D$(c=1, $CHCl_3$) = + 4.2°; MS = 357, 342, 330 and 302 m/e; NMR ($CDCl_3$) = 0.93, 1.10, 3.3, 3.97 and 5.3 δ

Example 4 17β-Cyano-17α-(α-ethoxyethyl)-etherandrost-5-en-3-ethylidene ketal (IIAb)

A mixture of 17β-cyano-17α-hydroxyandrost-5-en-3-ethylidene ketal (IAb, Example 3, 3.57 g) in methylene chloride (10 ml) containing ethyl vinyl ether (2 ml) and pyridine hydrochloride (100 mg) is heated in a sealed bottle at 40° for 2.5 hours and at 40° for 4 hours. Triethylamine (0.2 ml) is added and the reaction washed with phosphate buffer (pH 7), water and then dried over sodium sulfate, filtered and the filtrate concentrated under reduced pressure to give the title compound (4.61 g) as an oily mixture of diastereomers; NMR ($CDCl_3$) = 0.98, 1.04, 3.5, 3.94, 5.0 and 5.2 δ

Example 5 17α-(α-ethoxyethyl)-ether-20-acetyliminopregn-5-en-3-ethylidene ketal (IIIAb)

Methyl lithium (1.5 M, 1.2 ml) is added to a mixture of 17β-cyano-17α-(α-ethoxyethyl)-ether-androst-5-en-3-ethylidene (IIAb, Example 4, 550 mg) and toluene (3.5 ml) at 0°. After 2.5 hours at 0° the reaction is quenched by adding acetic anhydride (1 ml) and toluene (1 ml) resulting in a stirrable gel. Aqueous phosphate buffer (pH 7) is added, the phases are separated and the the lower aqueous phase is backwashed with ethyl acetate. The combined organic phases are backwashed with sodium bicarbonate (5%), phosphate buffer, dried over sodium sulfate and concentrated to give the title compound (643 ml) as an oil; NMR ($CDCl_3$) = 0.71, 1.02, 1.92, 2.00, 2.12, 3.4, 3.93, 4.7 and 5.3 δ; MS = 487, 415 and 297 m/e

Example 6 17α-(α-ethoxyethyl)-ether-20-acetylaminopregna-5,20-dien-3-ethylideneketal (VAb)

17α-(α-ethoxyethyl)-ether-20-acetylaminopregna-5-en-3-ethylidine ketal (IIIAb, Example 5, 3 mM) is stirred in glacial acetic acid (2 ml) for 1.5 hour at 20-25°. Toluene is added and the organic diluents

removed under reduced pressure. Additional toluene is added and the mixture washed with sodium bicarbonate (5%), phosphate buffer and the organic phase is dried over sodium sulfate and concentrated under reduced pressure to give the title compound (1.7 g) as an oil, NMR ($CDCl_3$) = 0.69, 1.02, 2.03, 3.4, 3.94, 4.6, 4.82, 4.97, 5.3, 5.84, 6.03 and 6.8 6

Example 7 17α-(α-ethoxyethyl)-ether-20-acetylaminopregna-5,20-dien-3-ethylideneketal (VAb)

Methyl magnesium bromide in ether (2.85 M, 7 ml) is added to a mixture of 17β-cyano-17α-(α-ethoxyethyl)-etherandrost-5-en-3-ethylidine ketal (IIAb, Example 4, 10 mM) in toluene (25 ml) and the mixture heated at 55° for 6 hours in a sealed pressure bottle. The mixture is then dissolved in THF (10 ml) and is added to toluene (20 ml) containing acetic anhydride (3 ml) which had been previously cooled to 0°. This mixture is allowed to warm to 25° during a 1/2 hour stirring period after which it is extracted with phosphate buffer (twice), dried over sodium sulfate, concentrated to give the title compound as an oil. This product is spectroscopically and chromatographically identical to the product of Example 6.

Example 8 17α-Acetoxy-20-acetylaminopregna-5,20-dien-3-ethylidene ketal (IVAb)

17β-Cyano-17α-(α-ethoxy ethyl)-ether androst 5-en-3-ethylidene ketal (VAb, Example 7) is dissolved in glacial acetic acid (10 ml) and acetic anhydride (5 ml) and the mixture stirred for 24 hours at 20-25°. The reaction mixture is then added to aqueous phosphate buffer and extracted with ethyl acetate. The phases are separated and the organic phase is washed with water, sodium bicarbonate (5%), water again, dried over sodium sulfate and concentrated to give the title compound as an oil; NMR ($CDCl_3$) = 0.73, 1.07, 2.08, 3.97, 5.00, 5.3, 5.8 and 6.6 δ; CMR ($CDCl_3$) = 169.78, 140.23, 138.85, 121.68, 109.35, 104.01, 93.59 and 64.36 δ; MS = 398 (m + 1); IR ($CHCl_3$) = 1730, 1685 $cm^{-1}$

Example 9 17α-Acetoxy-20-acetylaminopregna-5,20-dien-3-ethylidene ketal (IVAb)

Following the general procedure of Example 8 and making noncritical variations, but starting with 17α-(α-ethoxy ethyl)-ether-20-acetylaminopregn-5-en-3-ethylidene ketal (IIIAb, Example 5) the title compound is obtained as an oil identical in all respects to the product of Example 8.

Example 10 17α-Acetoxyprogesterone (VIA)

Acetic acid (1.5 ml) and water (0.5 ml) are added to 17α-acetoxy-20-acetylaminopregna-5,20-dien-3-ethylidene ketal (IVAb, Example 8, 0.5 mM) in acetic acid (0.66 ml) and acetic anhydride (0.34 ml). The reaction mixture is heated at 55° for approximately 18 hours. TLC (ethyl acetate/toluene:3/7) shows the hydrolysis to be complete to approximately a 90/10 mixture of 17α-acetoxyprogesterone and 17α-hydroxyprogesterone. Acetic anhydride (0.75 ml) and p-TSA (30 mg) are added and heating continued for 2 hours at 50°. Water (0.3 ml) is added and the heating is continued at 50° for 7.5 hours. Water (2 ml) is then added resulting in a precipitate which is filtered and dried to give the title compound.

Example 11 17R,2'-Methyl-4'-methylenespiro-3-ethylenedioxyandrost-5-en-17,5'(4'H)-oxazole (IXAb)

Acetic acid (5 ml) and acetic anhydride (0.4 ml) are added to 17α-(α-ethoxyethyl)-ether-20-acetylaminopregn-5-en-3-ethylidene ketal (IIIAb, Example 5, 10 mM) and the mixture heated to 55°. After 1 hour of heating at 55°, additional acetic acid (5 ml) and acetic anhydride (0.4 ml) are added and the heating continued for an additional 2.5 hours. The reaction mixture is cooled, the solid filtered, washed with diethyl ether and dried to give the title compound (1.45 g), m.p. = 199-209°; NMR ($CDCl_3$) = 0.81, 1.03, 2.04, 3.86, 4.65 and 5.30 δ; MS (CI) = 398 (M + 1)

Example 12 17R,2'-Methyl-4'-methylenespiro-3-ethylenedioxyandrost-5-en-17,5'(4'H)-oxazole (IXAb)

Methanol (2 ml) and p-TSA.$H_2O$ (2 ml) is added to 17α-(α-ethoxy ethyl)-ether-20-acetylaminopregn-5,20-dien-3-ethylidine ketal (VAb, Example 6, 200 ml) and the mixture heated to 70° for 40 minutes. Analysis by TLC and NMR shows that the product is mostly the title compound along with small amounts of 3-methyl enol ether and Δ4-3-keto analogues.

Example 13 17R,2'-Methyl-4'-bromomethylenespiro-3-ethylenedioxyandrost-5-en-17,5'(4'H)-oxazole (XAb)

Pyridium bromide perbromide (3 ml) is added to 17R,2'-methyl-4'-methylenespiro-3-ethylenedioxyandrost-5-en-17,5'(4'H)-oxazole (IXAb, Example 11, 50 ml) in methylene chloride (1 ml) and pyridine (0.05 ml) at 0°. TLC shows a single less polar product. A few drops of sodium bisulfite (1 M) and acetic acid are added. The phases are separated and the organic phase is washed with water, dried over sodium sulfate, and concentrated to give the title compound, NMR ($CDCl_3$) = 0.92, 1.03, 2.17, 3.93, 5.27 and 5.33 δ; MS ($CI,CH_4$) = 476, 478 (M + 1), 396

Example 14 21-Bromo-17α-acetoxyprogesterone (XIA)

17R,2'-Methyl-4'-bromomethylenespiro-3-ethylenedioxyandrost-5en-17,5'(4'H)-oxazole (XAb, Example 13). The product from Example 13 is dissolved in a solution of acetic acid (0.75 ml) and water (0.25 ml) and heated overnight at about 105° in a sealed vial. The reaction mixture is then added dropwise to a solution of phosphate buffer (pH 7) giving the title compound, NMR ($CDCl_3$) = 0.72, 1.17, 2.10, 3.90 and 5.67 δ

Example 15 21-Bromo-17α-(α-ethoxyethyl)-ether-20-acetylaminopregna-5-en-3-ethylidine ketal (XVAb)

Pyridinium hydrobromide perbromide (0.29 g) is added to 17α-(α-ethoxy ethyl)-ether-20-acetylaminopregna-5,20-dien-3-ethylidine ketal (VAb, Example 6, 411 mg) in a mixture of methylene chloride (3 ml), methanol (0.75 ml), water (0.12 ml) and triethylamine (0.26 ml) at 0°. The reaction is complete after about 5 minutes. Methylene chloride and water are added, the phases separated, the organic phase is dried over sodium sulfate and concentrated to give the title compound, NMR ($CDCl_3$) = 0.77, 0.83, 1.03, 2.27, 2.29, 3.9 and 5.27 δ; MS (CI) = 566 and 568 (m + 1) and 550.

Example 16 17α,21-Dihydroxypregn-4-en-3,20-dione 17,21-diacetate (XIIA)

21-Bromo-17α-acetoxyprogesterone (XIA, Example 14, 50 mg) is dissolved in toluene (0.3 ml) and DMF (0.3 ml) with acetic acid (4 µl) and stirred with potassium acetate (19 mg) at 70° in a sealed vial for 22.5 hours. Additional toluene is added and the mixture extracted with water. The organic phase is dried over sodium sulfate and concentrated under reduced pressure to give the title compound: NMR ($CDCl_3$) 0.76, 1.20, 2.07. 2.13, 4.55, 5.85 and 5.67 δ.

Example 17 21-Bromo-17α-hydroxyprogesterone (XIIIA)

Sulfuric acid (1N, 1 drop) is added to 21-bromo-17α-(α-ethoxyethyl)-ether-20-acetylaminopregn-5-en-3-ethylidine ketal (XVAb, Example 15, 50 mg) in THF (0.6 ml) and water (0.2 ml). This mixture is stirred at 20-25° for 18 hours and then heated at 60° for 17.5 hours to give the title compound; NMR ($CDCl_3$) = 0.73, 1.20, 4.22 and 5.70 δ.

Example 18 17α-(α-ethoxyethyl)-ether-20-trifluoroacetyl-acetylaminopregn-5-ene 3-ethylidene ketal (IIIAb)

A solution of 17β-cyano-17α-(α-ethoxyethyl)-etherandrost-5-en-3-ethylidene ketal (IIAb, Example 4, 3.5 mmole) in toluene (12.5 ml) is cooled to 0°. Methyllithium (1.6 M, 2.95 ml) in diethyl ether is added (dropwise) over a period of 4 hours. The reaction is stirred an additional 3 hours at 0° and then quenched at -35° with pyridine (0.76 ml) and trifluoroacetic hydride (1 ml). The reaction mixture is extracted 3 times with phosphate buffer (pH 7), the organic layer is dried over sodium sulfate and concentrated to give the title compound: NMR ($CDCl_3$) 0.71, 1.01, 1.20, 2.05, 2.11, 3.40, 3.90, 4.63 and 5.28 δ.

Example 19 17α-(α-Ethoxyethyl)-ether-20-trifluoroacetylaminopregna-5,20-diene 3-ethylidene ketal (VAb)

DBU (50 µl, 0.3 mmole) is added to 17α-(α-ethoxyethyl)-ether-2-trifluoroacetylaminopregna-5-ene-3-ethylidene ketal (IIIAb, Example 18, 3.5 mmole) in toluene (10 ml) and stirred at 20-25° for 70 minutes. The mixture is then extracted 3 times with phosphate buffer (pH 7), dried over sodium sulfate and concentrated to an oil: NMR ($CDCl_3$) 0.70, 1.03, 1.23, 3.47, 3.90, 4.70, 5.04, 5.18, 5.29, 5.88 and 6.17 δ.

Example 20 21-Bromo-17α-(α-ethoxyethyl)-ether-20-trifluoroacetylaminopregn-5-ene 3-ethylidene ketal (XVAb) and 21-bromo-17α-(α-ethoxyethyl)-ether-20-acetylaminopregna-5,20-diene 3-ethylidene ketal (XXAb)

17α-(α-Ethoxyethyl)-ether-20-trifluoroacetylaminopregna-5,20-diene-3-ethylidene ketal (VAb, Example 19, 190 mg) is dissolved in methylene chloride (0.5 ml) and methanol (0.125 ml) with triethylamine (61 µl) and water (20 µl). The mixture is cooled to 0°. Pyridinium hydrobromide perbromide (0.105 g) is added incremently. The reaction mixture is stirred at 0° for 2.2 hours and then extracted with phosphate buffer (pH 7). The organic layer is dired over sodium sulfate and concentrated to give a mixture of the title compounds: NMR ($CDCl_3$) 0.71, 0.75, 0.82, 1.03, 1.27, 3.43, 3.93, 4.33, 4.70, 5.3, 6.47 and 6.53 δ.

Example 21 21-Bromo-17α-hydroxyprogesterone (XIIIA) and 21-Bromo-17α-trifluoroacetylprogesterone(XIA)

The mixture of XVAb and XXAb (Example 20) as an oil (110 ml) is dissolved in THF (1.2 ml) with sulfuric acid (1 N, 0.2 ml). The mixture is heated at 54° for 9 hours. The mixture is extracted into ethyl acetate, and washed with phosphate buffer (pH 7) to neutrality. The mixture is then concentrated to an oil which, as shown by NMR analysis, contains a mixture of the title compounds.

Example 22 21-Bromo-17α-hydroxyprogesterone (XIIIA)

The mixture of compounds XIIIA and XIA (Example 21) as an oil (160 mg) is dissolved in methanol (6.0 ml) and water (1 ml) with sodium bicarbonate (172 mg). The mixture is stirred at 20-25° for 2 hours. The mixture is extracted with methylene chloride, and the methylene chloride extract is concentrated to give the title compound: NMR ($CDCl_3$) 0.73, 1.20, 4.09, 4.37 and 5.69 δ.

Example 23 17α-(α-Ethoxyethyl)-ether-20-trimethylacetylaminopregna-5,20-diene 3-ethylidene ketal (VAb)

A 2:1 mixture of 17α-(α-ethoxyethyl)-ether-20-trimethylacetylaminopregna-5,20-diene 3-ethylidene ketal (VAb) and 17α-(α-ethoxyethyl)-ether-20-trimethylacetylaminopregn-5-ene 3-ethylidene ketal (IIIAb) is dissolved in toluene (5 ml) and THF (1 ml) and stirred with several mg of magnesium bromide under nitrogen at 20-25°. After 16 hr, all of (IIIAb) is epimerised to (VAb).

CHART A

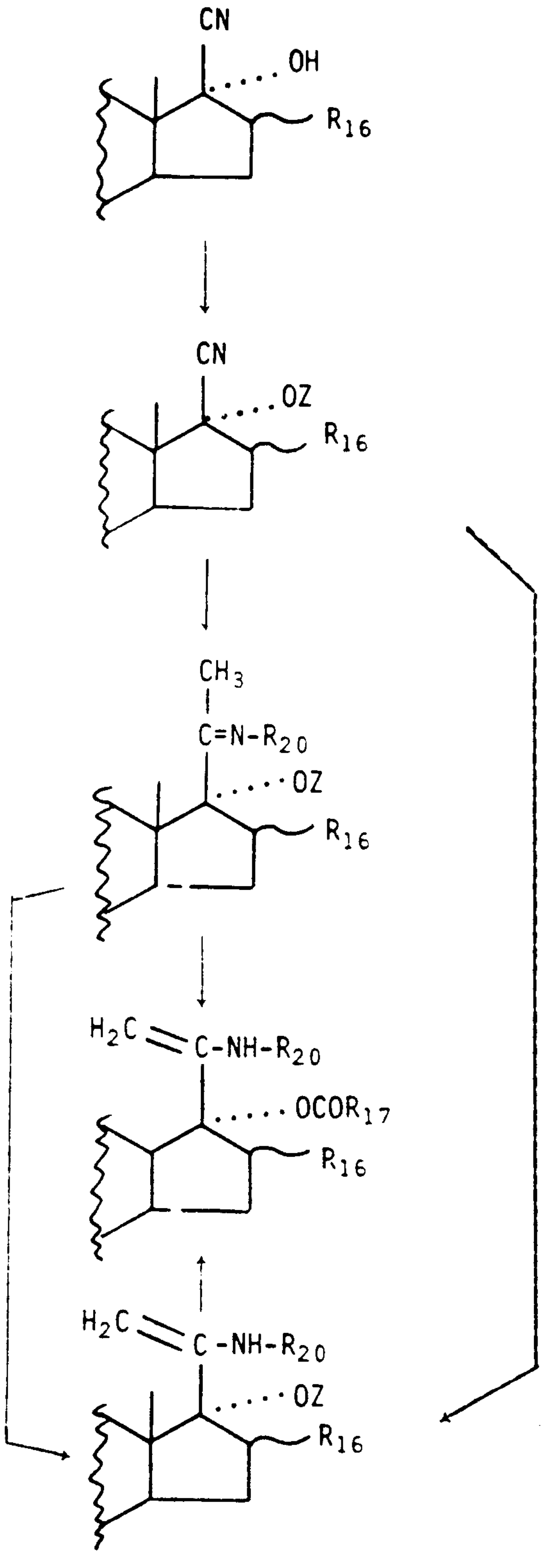

(I)

(II)

(III)

(IV)

(V)

CHART B

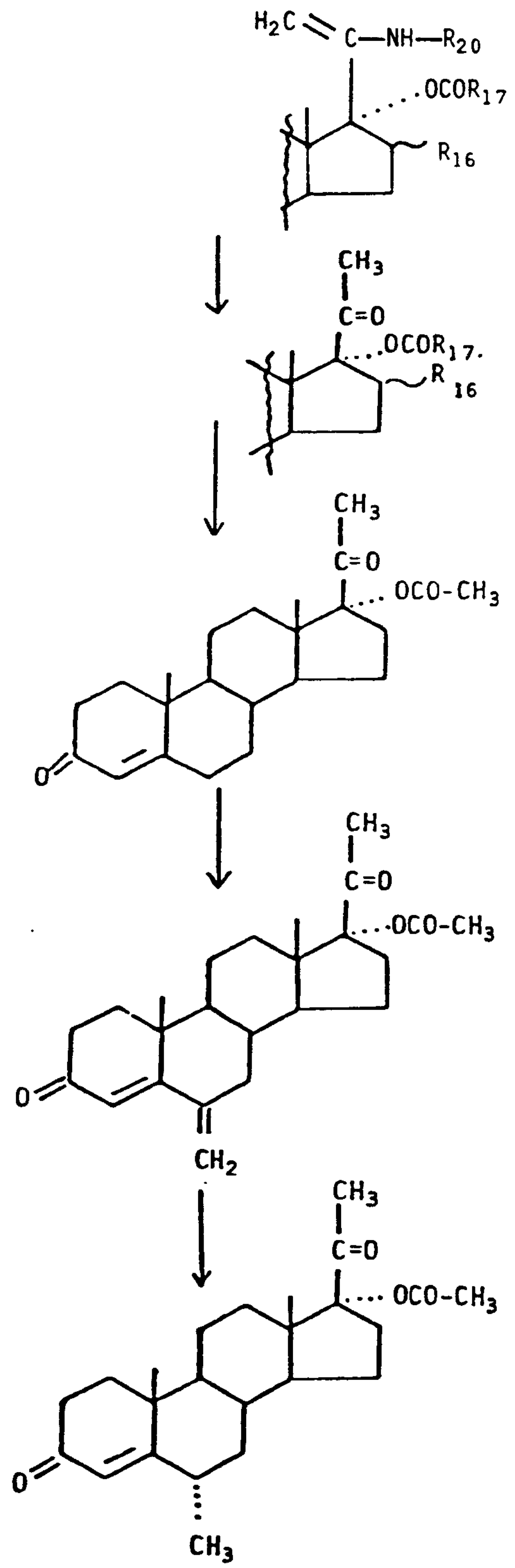

(IV)

(VI)

(VI'A)

(VII)

(VIII)

## CHART C

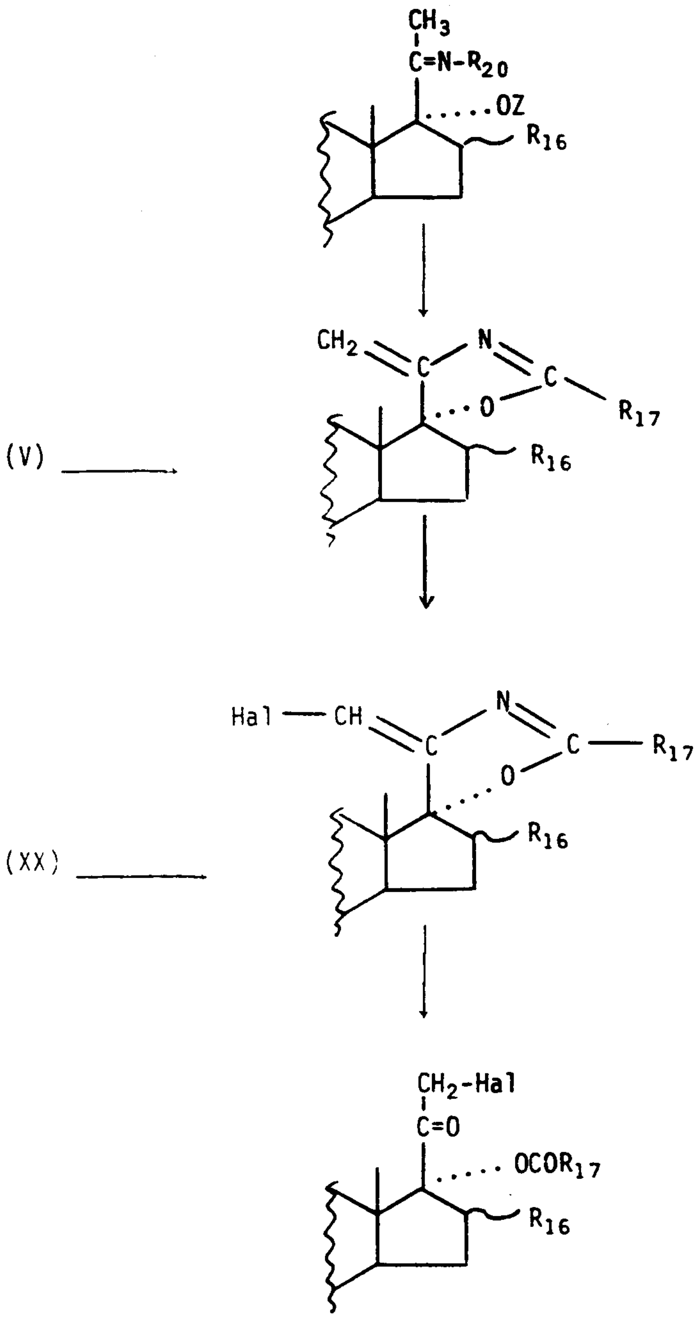


(III)

(IX)

(X)

(XI)

CHART D

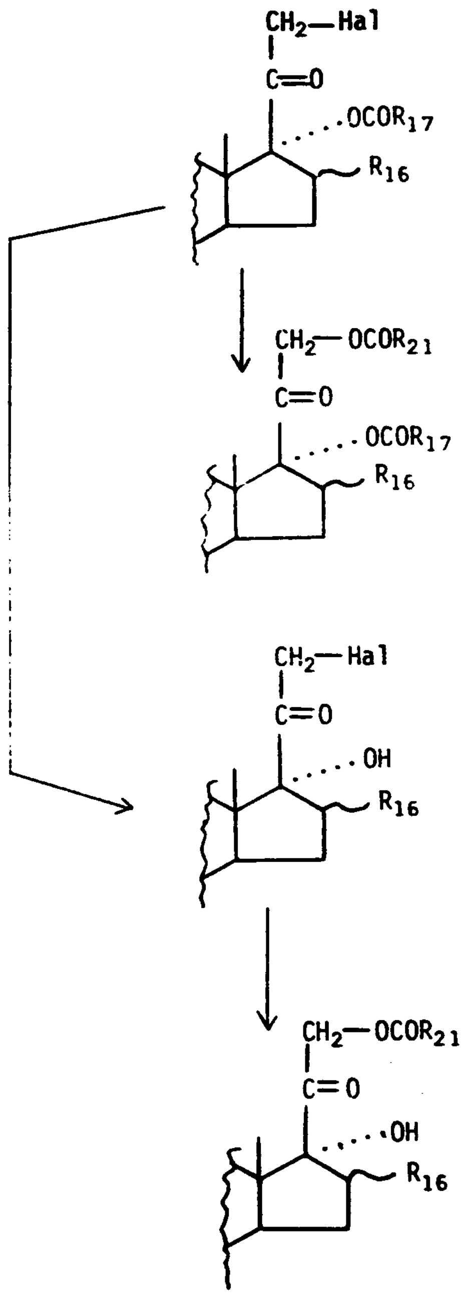

(XI)

(XII)

(XIII)

(XIV)

CHART E

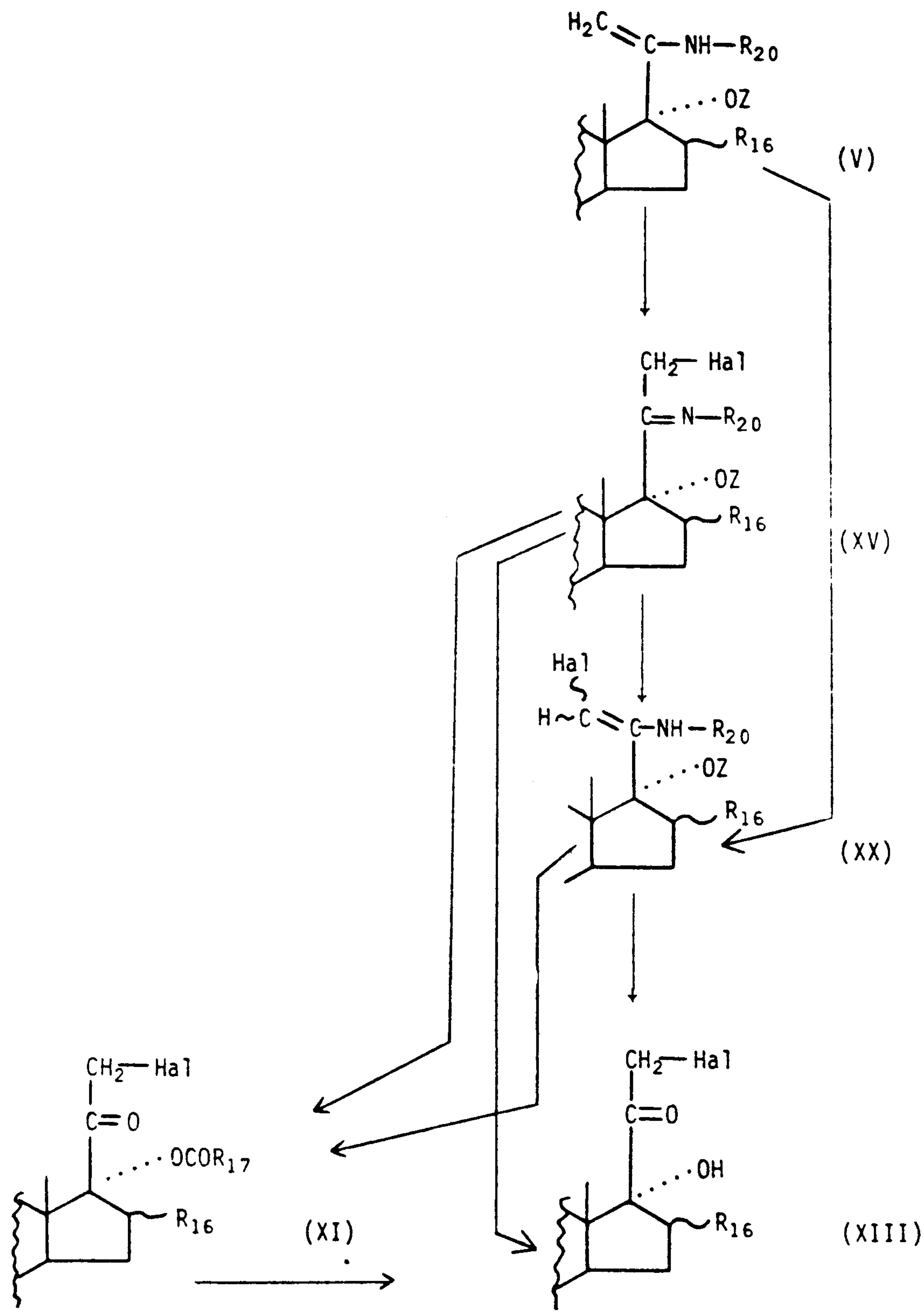

(V)

(XV)

(XX)

(XI)

(XIII)

CHART F

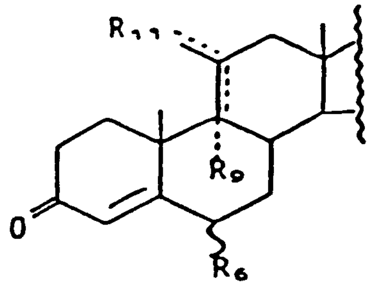

(A)

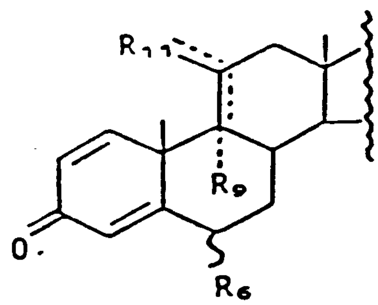

(B)

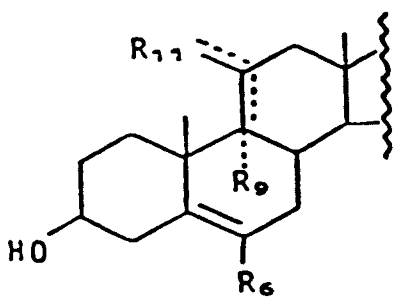

(C)

CHART G

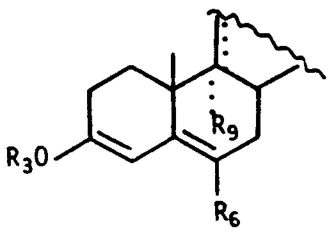

(Aa)

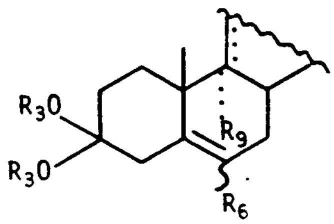

(Ab)

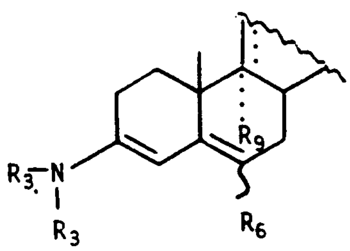

(Ac)

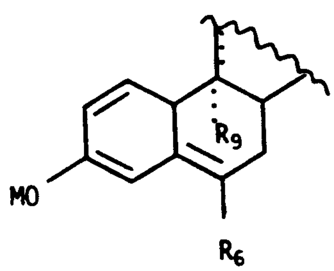

(Ba)

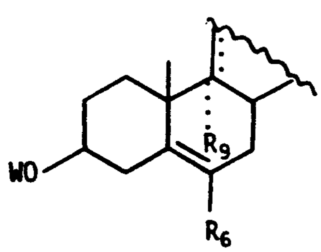

(Ca)

## Claims

**1.** An enimide of formula III A-C or $\Delta^{20}$-enamide of formula V A-C

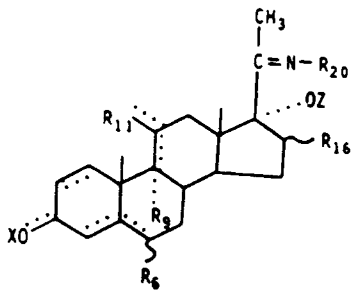

(III A-C)

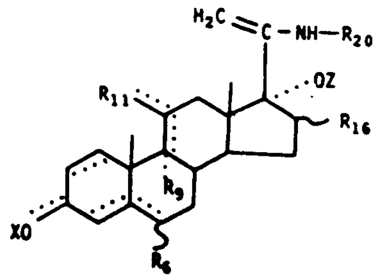

(V A-C)

or a $C_3$-protected form thereof, wherein

~ indicates $\alpha$ or $\beta$ configuration;

... is a single or double bond;

...OX is =O or -OH;

$R_6$ is H, F or $CH_3$;

either $R_9$ is absent, $R_{11}$ is H, and the C-ring is $\Delta^{9(11)}$; $R_9$ and $R_{11}$ together form a 9$\beta$,11-epoxide; or $R_9$ is H or F and ...$R_{11}$ is -H, ~OH or =O;

$R_{16}$ is H or $CH_3$;

$R_{20}$ is $-Si(CH_3)_3$, $-Si(CH_3)_2C(CH_3)_3$ or $-OC-R_{20}'$ in which $R_{20}'$ is $C_{1-5}$ alkyl or phenyl substituted by 0 to 2 Cl, $CH_3$ or $NO_2$ substituents; and

Z is $-Si(CH_3)_3$, $-Si(CH_3)_2(CH_3)_3$, tetrahydropyranyl, $-CH_2OCH_3$ or ethoxyethyl.

**2.** A process for the preparation of a $C_3$-protected form of an enimide of formula III A-C as claimed in claim 1, which comprises the steps of:

(1) contacting a corresponding $C_3$-protected form of a 17$\beta$-cyano steroid of formula II A-C

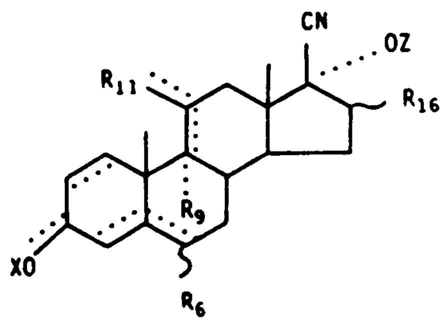

(II A-C)

with a methylating agent; and

(2) contacting the product of step (1) with an appropriate acylating agent of the formula $R_{20}'COHal$ or $(R_{20}'CO)_2O$, or with an appropriate silylating agent of the formula $(CH_3)_3SiHal$ or $(CH_3)_3C(CH_3)_2SiHal$.

**3.** A process according to claim 2, which comprises the additional prior steps of:

(1) reacting a 17-keto steroid corresponding to the 17-protected-17$\beta$-cyano-17$\alpha$-hydroxy steroid of formula II A-C with potassium cyanide and acetone cyanohydrin in an alcoholic solvent, and

(2) introducing into the resultant 17-unprotected-17$\beta$-cyano-17$\alpha$-hydroxy steroid the protecting group Z, by reaction with a suitable etherifying reagent using an acid catalyst, or in an inert solvent; and

(3) introducing a $C_3$-protecting group before or after step (2).

**4.** A process according to claim 2 or claim 3, which additionally comprises reacting the product of formula III A-C with a carboxylic acid of the formula $R_{17}COOH$ and an anhydride of the formula $R_{17}(CO)_2O$, wherein $R_{17}$ is H, $C_{1-5}$ alkyl, $CCl_3$, $CF_3$ or phenyl substituted by 0 to 2 Cl atoms, thereby obtaining a $C_3$-protected form of a corresponding $\Delta^{20}$-enamide acylate of formula IV A-C

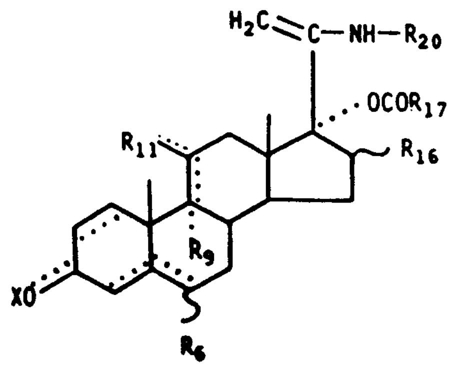

(IV A-C)

5. A process according to claim 2 or claim 3, which additionally comprises reacting the product with an acid in an organic solvent, thereby obtaining a $C_3$-protected form of a corresponding oxazoline of formula IX A-C

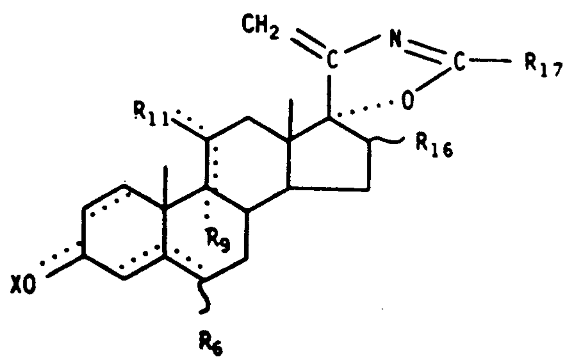

(IX A-C)

6. A process for the preparation of a $C_3$-protected form of a 21-haloenimide of formula XV A-C or 21-halo-$\Delta^{20}$-enimide of formula XX A-C

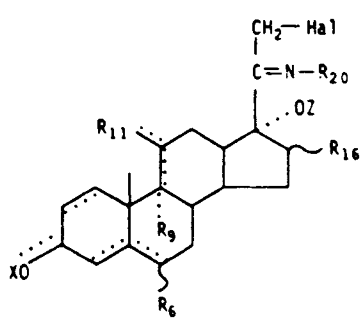

(XV A-C)

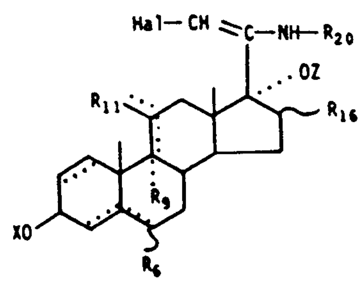

(XX A-C)

wherein Hal is Cl, Br or I and ~, ..., ...OX, $R_6$, $R_9$, $R_{11}$, $R_{16}$, $R_{20}$ and Z are as defined in claim 1, which comprises contacting a $C_3$-protected form of a $\Delta^{20}$-enimide of formula V A-C as claimed in claim 1 with a halogenating agent.

7. A process according to claim 6, which comprises the prior steps of a process according to claim 2 or claim 3 and tautomerisation of the product of formula III A-C to the $\Delta^{20}$-enimide of formula V A-C.

8. A process according to claim 6 or claim 7, comprising the additional step of reacting the product of formula XV A-C or XX A-C with an (aqueous) acid, thereby obtaining a $C_3$-protected form of a corresponding 17$\alpha$-hydroxy steroid of formula XIII A-C

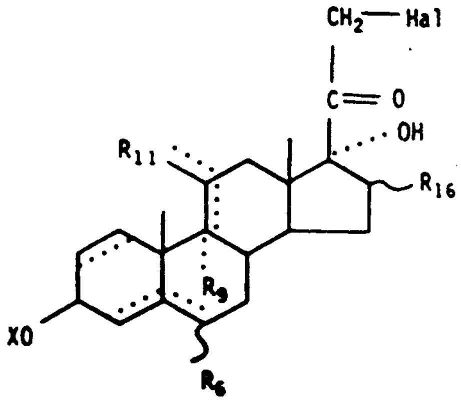

(XIII A-C)

9. A process according to any of claims 6 to 8, wherein the halogenating agent is bromine, pyridinium hydrobromide perbromide, N-bromosuccinimide, N-chlorosuccinimide or dibromantin.

**Revendications**

1. Enimide de formule III A-C ou $\Delta^{20}$-énamide de formule V A-C

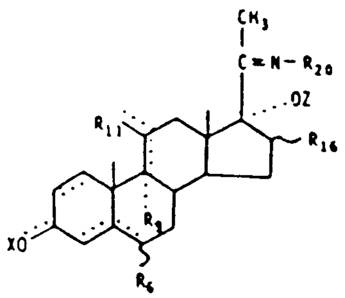

(III A-C)

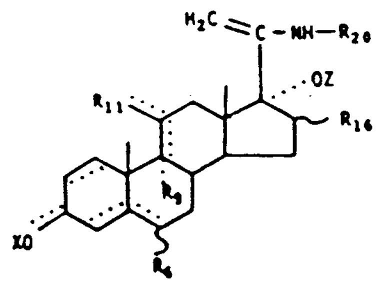

(V A-C)

ou leur forme protégée en $C_3$, formules dans lesquelles

~indique la configuration $\alpha$ ou $\beta$;

... indique une liaison simple ou double ;

$\overline{...}$OX représente =O ou -OH ;

$\overline{R_6}$ représente H, F ou $CH_3$ ;

$R_9$ est absent, $R_{11}$ représente H et le noyau C est un noyau $\Delta^{9(11)}$ ; $R_9$ et $R_{11}$ forment ensemble un 9$\beta$,11-époxyde; ou bien $R_9$ représente H ou F et $\underline{...}R_{11}$ représente -H,~OH ou =O ;

$R_{16}$ représente H ou $CH_3$ ;

$R_{20}$ représente $-Si(CH_3)_3$, $-Si(CH_3)_2C(CH_3)_3$ ou -OC-$R_{20}$' où $R_{20}$' est un groupe alkyle en $C_1$ à $C_5$ ou un groupe phényle portant 0 à 2 substituants Cl, $CH_3$ ou $NO_2$ ; et

Z est un groupe $-Si(CH_3)_3$, $-Si(CH_3)_2C(CH_3)_3$, tétrahydropyrannyle, $-CH_2OCH_3$ ou éthoxyéthyle.

2. Procédé de préparation d'une forme protégée en $C_3$ d'un énimide de formule III A-C suivant la revendication 1, qui comprend les étapes consistant :

(1) à faire entrer une forme protégée en $C_3$ correspondante d'un 17$\beta$-cyano-stéroïde de formule II A-C

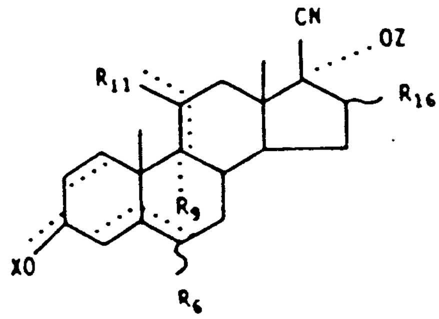

(II A-C)

en contact avec un agent de méthylation ; et

(2) à faire entrer le produit de l'étape (1) en contact avec un agent acylant approprié de formule $R_{20}$'COHal ou $(R_{20}'CO)_2O$ ou avec un agent de silylation approprié de formule $(CH_3)_3SiHal$ ou $(CH_3)_3C(CH_3)_2SiHal$.

3. Procédé suivant la revendication 2, qui comprend les étapes préalables additionnelles consistant :

(1) à faire réagir un 17-céto-stéroïde correspondant au 17$\beta$-cyano-17$\alpha$-hydroxy-stéroïde protégé en 17 de formule II A-C avec du cyanure de potassium et de la cyanhydrine acétonique dans un solvant alcoolique, et

(2) à introduire dans le 17$\beta$-cyano-17$\alpha$-hydroxy-stéroïde non protégé en 17 le groupe protecteur Z par réaction avec un réactif convenable d'éthérification en utilisant un catalyseur acide ou dans un solvant inerte ; et

(3) à introduire un groupe protégeant la position $C_3$ avant ou après l'étape (2).

4. Procédé suivant la revendication 2 ou la revendication 3, qui consiste en outre à faire réagir le produit de formule III-A-C avec un acide carboxylique de formule $R_{17}COCH$ et un anhydride de formule $R_{17}$-$(CO)_2O$ où $R_{17}$ représente H, un groupe alkyle en $C_1$ à $C_5$, $CCl_3$, $CF_3$ ou phényle substitué par 0 à 2 atomes de chlore, de manière à obtenir une forme protégée en $C_3$ d'un acylate de $\Delta^{20}$-énamide correspondant de formule IV A-C

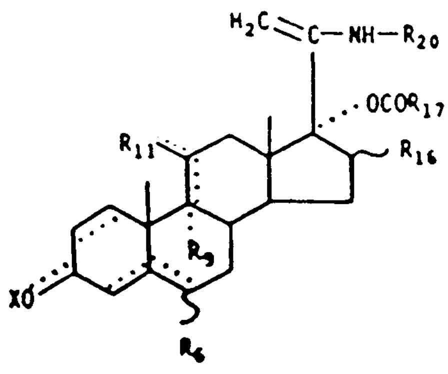

(IV A-C)

5. Procédé suivant la revendication 2 ou la revendication 3, qui comprend en outre la réaction du produit avec un acide dans un solvant organique de manière à produire une forme protégée en $C_3$ d'une oxazoline correspondante de formule IX A-C

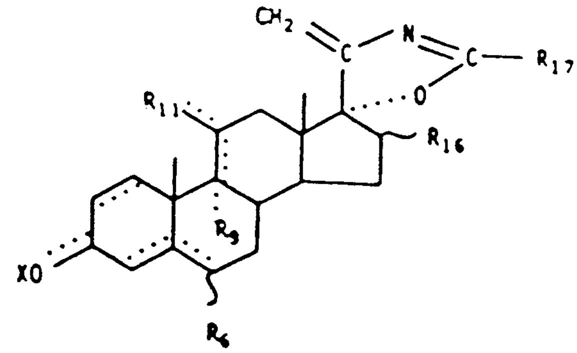

(IX A-C)

6. Procédé de préparation d'une forme protégée en $C_3$ d'un 21-halogéno-énimide de formule XV A-C ou d'un 21-halogéno-$\Delta^{20}$-énimide de formule XX A-C

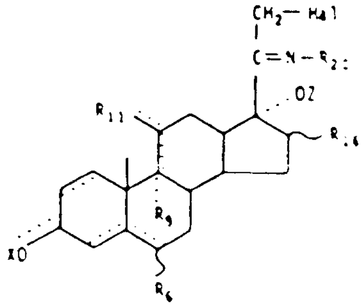

(XV A-C)

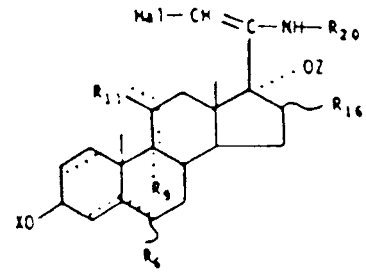

(XX A-C)

formules dans lesquelles Hal représente Cl, Br ou I et ~, ..., ...OX, $R_6$, $R_9$, $R_{11}$, $R_{16}$, $R_{20}$ et Z sont tels que définis dans la revendication 1, qui consiste à faire entrer une forme protégée en $C_3$ d'un $^{20}$-énimide de formule V A-C suivant la revendication 1 en contact avec un agent d'halogénation.

7. Procédé suivant la revendication 6, qui comprend les étapes préalables d'un procédé suivant la revendication 2 ou la revendication 3 et la tautomérisation du produit de formule III A-C en le $\Delta^{20}$-énimide de formule V A-C.

8. Procédé suivant la revendication 6 ou la revendication 7, qui comprend l'étape additionnelle de réaction du produit de formule XV A-C ou XX A-C avec un acide (aqueux) de manière à obtenir une forme protégée en $C_3$ d'un 17α-hydroxy-stéroïde correspondant de formule XIII A-C

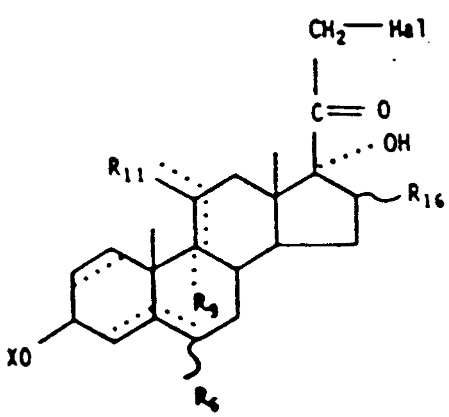

(XIII A-C)

9. Procédé suivant l'une quelconque des revendications 6 à 8, dans lequel l'agent d'halogénation est le brome, le perbromure de bromhydrate de pyridinium, le N-bromosuccinimide, le N-chlorosuccinimide ou la dibromantine.

## Patentansprüche

1. Enimid der Formel III A-C oder [20]-Enamid der Formel V A-C

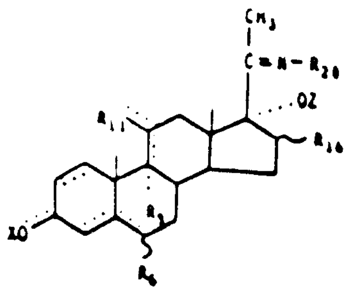

(III A-C)

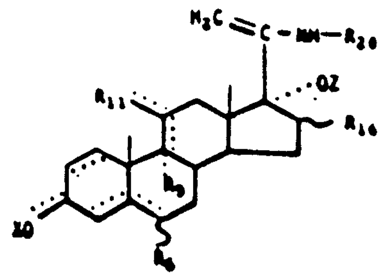

(V A-C)

oder eine $C_3$-geschützte Form davon, worin
~ $\alpha$- oder $\beta$-Konfiguration anzeigt;
... eine Einfach- oder Doppelbindung ist.
...OX = O oder -Oh ist;
$R_6$ H, F oder $CH_3$ ist;
entweder $R_9$ fehlt, $R_{11}$ H ist, und der C-Ring $\Delta^{9(11)}$ ist; $R_9$ und $R_{11}$ zusammen ein 9$\beta$,11-Epoxid bilden; oder $R_9$ H oder F ist und ...$R_{11}$ -H, OH oder =O ist;
$R_{16}$ H oder $CH_3$ ist;
$R_{20}$ $-Si(CH_3)_3$, $-Si(CH_3)_2C(CH_3)_3$ oder -OC-$R_{20}$' ist, wobei $R_{20}$' $C_{1-5}$Alkyl oder Phenyl ist, substituiert durch 0 bis 2 Cl-, $CH_3$- oder $NO_2$-Substituenten; und
Z $-Si(CH_3)_3$, $-Si(CH_3)_2C(CH_3)_3$, Tetrahydropyranyl, $-CH_2OCH_3$ oder Äthoxyäthyl ist.

2. Verfahren zur Herstellung einer $C_3$-geschützten Form eines Enimids der Formel III A-C nach Anspruch 1, das die folgenden Schritte umfasst:
(1) eine entsprechende Form eines 17$\beta$-Cyanosteroids der Formel II A-C

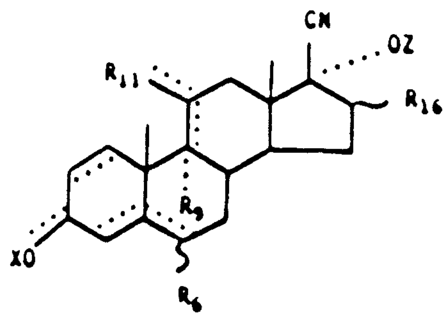

(II A-C)

mit einem methylierenden Mittel in Kontakt zu bringen; und
(2) das Produkt von Schritt (1) mit einem geeigneten acylierenden Mittel der Formel $R_{20}$'COHal oder ($R_{20}$'CO)$_2$O, oder mit einem geeigneten silylierenden Mittel der Formel $(CH_3)_3SiHal$ oder $(CH_3)_3C$-$(CH_3)_2SiHal$ in Kontakt zu bringen.

3. Verfahren nach Anspruch 2, das die zusätzlichen vorhergehenden folgenden Schritte umfasst:
(1) ein 17-Ketosteroid, das dem 17-geschützten-17$\beta$-Cyano-17$\alpha$-hydroxysteroid der Formel II A-C entspricht, mit Kaliumcyanid und Acetoncyanhydrin in einem alkoholischen Lösungsmittel zur Reaktion zu bringen, und
(2) in das resultierende 17-ungeschützte-17$\beta$-Cyano-17$\alpha$-hydroxysteroid die schützende Gruppe Z, durch Reaktion mit einem geeigneten veräthernden Reaktionsmittel, das einen Säurekatalyst benutzt, oder in einem inerten Lösungsmittel, einzuführen; und
(3) eine $C_3$-schützende Gruppe vor oder nach Schritt (2) einzuführen.

4. Verfahren nach Anspruch 2 oder Anspruch 3, das zusätzlich umfasst, das Produkt der Formel III A-C mit einer Kohlenstoffsäure der Formel $R_{17}COOH$ und einem Anhydrid der Formel $R_{17}(CO)_2O$ zur

Reaktion zu bringen, worin $R_{17}$ H ist, $C_{1-5}$ Alkyl, $CCl_3$, $CF_3$, oder Phenyl, substituiert durch 0 bis 2 Cl-Atome, dabei wird eine $C_3$-geschützte Form eines entsprechenden $\Delta^{20}$-Enamidacylates der Formel IV A-C

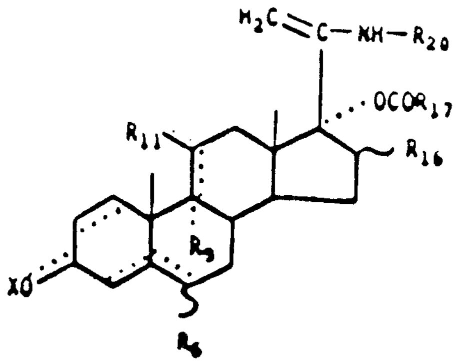

(IV A-C)

erhalten.

**5.** Verfahren nach Anspruch 2 oder Anspruch 3, das zusätzlich umfasst, das Produkt mit einer Säure in einem organischen Lösungsmittel zur Reaktion zu bringen, und damit eine $C_3$-geschützte Form eines entsprechenden Oxazolins der Formel IX A-C

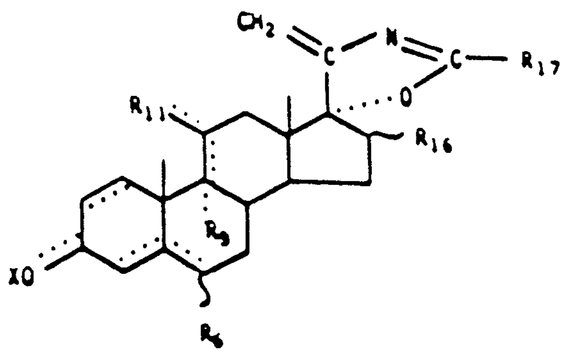

(IX A-C)

zu erhalten.

**6.** Verfahren zur Herstellung einer $C_3$-geschützten Form eines 21-Haloenimids der Formel XV A-C oder 21-Halo-$\Delta^{20}$-enimids der Formel XX A-C

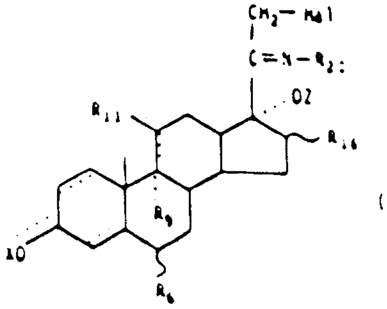

(XV A-C)

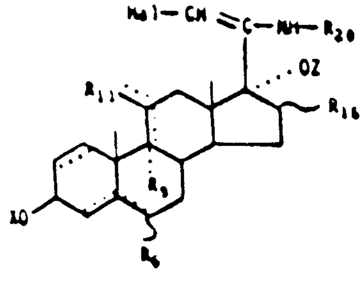

(XX A-C)

worin Hal Cl, Br oder I ist, und ~, ..., ...OX, $R_6$, $R_9$, $R_{11}$,$R_{16}$, $R_{20}$ und Z wie in Anspruch 1 definiert sind, das umfasst, eine $C_3$-geschützte Form eines $\Delta^{20}$-Enimids der Formel V A-C mit einem halogenierenden Mittel wie in Anspruch 1 in Kontakt zu bringen.

**7.** Verfahren nach Anspruch 6, das die vorhergehenden Schritte eines Verfahrens nach Anspruch 2 oder Anspruch 3 und Tautomerisierung des Produktes der Formel III A-C zu dem $\Delta^{20}$-Enimid der Formel V A-C umfasst.

8. Verfahren nach Anspruch 6 oder Anspruch 7, das den zusätzlichen Schritt umfasst, das Produkt der Formel XV A-C oder XX A-C mit einer (wässrigen) Säure zur Reaktion zu bringen, dabei eine $C_3$-geschützte Form eines entsprechenden 17α-Hydroxysteroids der Formel XIII A-C

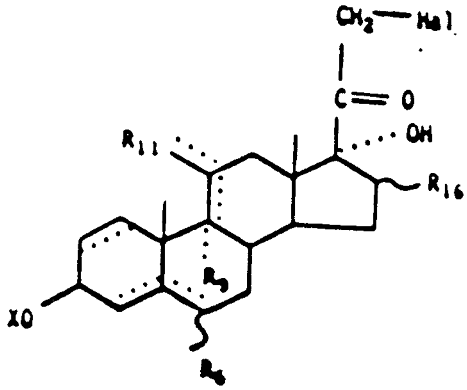

(XIII A-C)

zu erhalten.

9. Verfahren nach einem der Ansprüche 6 bis 8, worin das halogenierende Mittel Bromin, Pyridiniumhydrobromidperbromid, N-Bromosuccinimid, N-Chlorosuccinimid oder Dibromantin ist.